(19) Europäisches Patentamt
European Patent Office
Office européen des brevets

(11) Publication number: **0 172 318 B1**

(12) **EUROPEAN PATENT SPECIFICATION**

(45) Date of publication of patent specification: **24.07.91**

(51) Int. Cl.5: **C12P 1/06**, C07G 11/00, A61K 35/66, C12N 1/20, //(C12P1/06,C12R1:01), (C12N1/20,C12R1:01)

(21) Application number: **85105531.9**

(22) Date of filing: **07.05.85**

(54) **Antibiotic LL-C19004.**

(30) Priority: **25.06.84 US 624449**

(43) Date of publication of application:
**26.02.86 Bulletin 86/09**

(45) Publication of the grant of the patent:
**24.07.91 Bulletin 91/30**

(84) Designated Contracting States:
**AT BE CH DE FR GB IT LI NL SE**

(73) Proprietor: **AMERICAN CYANAMID COMPANY**
**1937 West Main Street P.O. Box 60**
**Stamford Connecticut 06904-0060(US)**

(72) Inventor: **Kirby, Jane Parsons**
**5 Henry Street**
**New City New York 10956(US)**
Inventor: **Labeda, David Paul**
**2320 West Pintura Court**
**Peoria Illinois 61614(US)**
Inventor: **Borders, Donald Bruce**
**13 Heather Hill Lane**
**Suffern New York 10901(US)**
Inventor: **Lee, May Dean-Ming**
**19 Shuart Road**
**Monsey New York 10952(US)**
Inventor: **Maiese, William Michael**
**891 Sherwood Road**
**Bridgewater New Jersey 08807(US)**
Inventor: **Testa, Raymond Thomas**
**55 Rockledge Road**
**Cedar Grove New Jersey 07009(US)**

(74) Representative: **Wächtershäuser, Günter, Dr.**
**Tal 29**
**W-8000 München 2(DE)**

EP 0 172 318 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a new antibacterial agent designated LL-C19004, to its production by fermentation, to methods for its recovery and concentration from crude solutions and to processes for its purification. The present invention includes within its scope the antibacterial agent in dilute form, as a crude concentrate and in pure form. The effects of this new agent on specific microorganisms, together with its chemical and physical properties, differentiate it from previously described. antibacterial agents.

The structure of LL-C19004 is unknown at the present time. However, the antibiotic appears to be a weakly basic polysaccharide containing a chromophoric group. The physiochemical characteristics of antibiotic LL-C19004 are as follows:

(a) Typical elemental analysis (approximate): C, 46.88; H, 6.93; N: 4.08; S, 1.79;

(b) Ultraviolet absorption spectra, as shown in FIG. I:

$$\lambda_{max}^{H_2O}\ nm(E_{1cm}^{1\%})\ 214\ (64),\ 285(63),\ 311\ sh(48);\ \lambda_{max}^{0.1NHCl}\ 214\ (60),\ 285(59),\ 311\ sh(45);\ \lambda_{max}^{0.1N\ NaOH}\ 227(54),\ 260(64),\ 295(57),\ 348(25);$$

(c) Infrared absorption spectrum, as shown in FIG. 2 (KBr disk):

(d) Proton magnetic resonance spectrum, as shown in FIG. 3 ($D_2O$); and

(e) Optical rotation: $[\alpha]_D^{25}$ = -85°±2° (concentration about 0.5%, water).

## BRIEF DESCRIPTION OF THE DRAWINGS

FIG. 1 represents the ultraviolet absorption spectra of antibiotic LL-C19004 (100 mcg/ml).

FIG. 2 represents the infrared absorption spectrum of antibiotic LL-C19004 (K Br disk).

FIG. 3 represents the proton magnetic resonance spectrum of antibiotic LL-C19004 ($D_2O$).

## DETAILED DESCRIPTION OF THE INVENTION

Culture LL-C19004, which produces the novel antibacterial agent LL-C19004, is a natural selection isolate of a culture isolated from a soil sample collected in Puerto Llano. Spain. The culture was taxonomically characterized and identified as new species of the genus *Saccharothrix*, to be known as *Saccharothrix espanaensis* Labeda, sp. nov.

This new species is maintained in the culture collection of the Medical Research Division, American Cyanamid Company, Pearl River, New York as culture number LL-C19004. A viable culture of this new microorganism has been deposited with the ARS Culture Collections, Fermentation Laboratory, Northern Regional Research Center, U. S. Department of Agriculture, 1815 North University Street, Peoria, Illinois 61604, and has been added to its permanent collection. It has been assigned by such depository the strain designation NRRL 15764. Access to such culture, under strain designation NRRL 15764. during pendency of the instant application shall be available to one determined by the Commissioner of Patents and Trademarks to be entitled thereto under 37 C.F.R. §1.14 and 35 U.S.C. §122, and all restrictions on availabillity to the public of such culture will be irrevocably removed upon grant of a patent on the instant application.

Observations were made of the cultural, physiological and morphological features of culture LL-C19004 using methods detailed by Shirling, E. B. and D. Gottlieb, Methods for Characterization of Streptomyces Species. internat. J. Syst. Bacteriol., 16, 313-340 (1966); and Gordon, R. E., et al., Internat. J. Syst. Bacteriol., 24, 54-63 (1974). Media used in the study were selected from those recommended by Pridham, T. G., et al., A Selection of Media for Maintainance and Taxonomic Study of Streptomycetes. Antibiotics Ann., pp. 947-953, 1956/57; Gauze, G. T., et al., Problems in the Classification of Antagonistic Actinomycetes. State Publishing House for Medical Literature, Medgiz, Moscow, 1957: and Gordon, R. E., Ecology of Soil Bacteria. T. G. R. Gray and B. Parkinson, Eds., Liverpool Univ. Press, 1967 for the Taxonomic Study of Actinomycetes and Soil Bacteria. Chemical composition of the cell walls of the culture was determined using the method of Lechevalier, H. A., et al., Chemical Composition as a Criterion in the Classification of Actinomycetes. Adv. Appl. Microbiol., 14, 47-72 (1971). Phospholipid patterns were determined using the method of Lechevalier, M. P., et al., Chemotaxonomy of Aerobic Actinomycetes:Phospholipid Composition. Biochem. Syst. Ecol., 5, 249-260 (1977). Acid production from

carbohydrates was determined by the method of Gordon, et al., (vide supra). Carbohydrate utilization was determined by the method of Pridham, T. G. and D. Gottlieb, The Utilization of Carbon Compounds by Some Actinomycetales as an Aid for Species Determination. J. Bacteriol., 56, 107-114 (1948). Details are recorded in Tables I-VI and a general description of the culture is given below. Underscored descriptive colors are taken from Kelly, K. L. and D. B. Judd, Color. Universal Language and Dictionary of Names. Nat. Bur. Stand. (U. S.), Spec. Publ. 440, 1976, Washington, D. C., and the accompanying Inter-Society Color Council, National Bureau of Standards Centroid Color Charts.

Micromorphology

Spores are formed in very short straight chains on rudimentary aerial sporophores. They may also result from fragmentation of vegetative mycelia. The spores are ovoid in shape, about 0.6 to 0.8 micron by about 1.0 to 1.2 micron and have a substantially smooth surface.

Cell Wall Composition

Whole cell hydrolysates of this culture contain the meso-isomer of diaminopimelic acid and mannose and galatose as the characteristic sugars. The phosphalipid pattern was found to be characteristic for Type PIV, with glucosamine-containing phospholipids as the significant phospholipid type. This chemotaxonomic pattern of cell wall composition, whole cell sugar and phospholipid pattern is characteristic of the genus Saccharothrix.

Amount of Growth

Good growth observed on asparagine-dextrose agar, Bennett's agar, NZ-amine-starch-glucose agar (ATCC Medium 172), tomato paste-oatmeal agar and yeast extract-malt extract agar; moderate growth observed on Benedict's agar, calcium malate agar, inorganic salts-starch agar and oatmeal agar; poor growth observed on Gauze No. 1 agar.

Vegetative Mycelium

On media where good growth occurred, the vegetative mycelium was observed to be flat and waxy and generally yellow to orange-yellow to yellowish brown shades in color.

Aerial Mycelium and Spore Color

Aerial mycelia absent on most media; very sparse white mycelia when present.

Soluble Pigments

Absent on most media; yellowish pigment on glycerol asparagine agar and inorganic salts-starch agar; brownish pigments on asparagine dextrose agar, NZ-amine-starch-glucose agar, oatmeal agar, tomato paste-oatmeal agar; red-dish-brown pigment on yeast extract-malt extract agar.

Physiological Reactions

No melanin pigments on peptone-iron agar and tyrosine agar (ISP-7); strong peptonization of litmus milk; no proteolysis of nutrient gelatin; weak to no reduction of nitrate; no hydrolysis of tyrosine or xanthine; strong hydrolysis of adenine and hypoxanthine; no decomposition of urea; strong hydrolysis of esculin. Good utilization of fructose, glucose, mannose, sucrose and trehalose; moderate utilization of galactose, glycerol and maltose; poor utilization of salicin; no utilization of adonitol, arabinose, dulcitol, inositol, lactose, mannitol, melezitose, melibiose, raffinose, rhamnose, sorbitol and xylose was observed. Good acid production from fructose, galactose, glucose, glycerol, maltose, mannose, sucrose, trehalose and xylose; no acid produced from adonitol, arabinose, inositol, lactose, mannitol, melezitose, melibiose, raffinose, rhamnose, salicin and sorbitol. Utilization of organic acids by the method of Gordon, et al., (vide supra): utilization of lactate and malate; no utilization of benzoate, citrate, mucate and oxalate. Poor growth at 4°C and 45°C, moderate growth at 32°C and 37°C, and good growth at 28°C.

The biochemical composition pattern for culture, meso-diaminopimelic acid, mannose and galactose,

and phospholipid pattern-type PIV, absolutely identifies it as a member of the genus Saccharothrix. This culture differs from the type species Saccharothrix australiensis NRRL 11239 in several ways. Culture LL-C19004 does not hydrolyze tyrosine or make melanin soluble pigments, although it does hydrolyze adenine, which is not hydrolyzed by the type strain. Another major difference between these strains is the inability of the type strain to grow on the ISP carbohydrate test medium due to its inability to utilize ammonium as a nitrogen source. Culture LL-C19004 is able to use at least nine different carbohydrates with this basal medium. The gross colonial appearance of these two strains is also different, with S. australiense having brown to yellowish brown vegetative mycelium and LL-C19004 having mycelia with orange-yellow shades on the same media. In the light of the significant differences between these two cultures, culture LL-C19004 is designated as a new species to be known as Saccharothrix espanaensis Labeda. being named for the collection site (country) of the soil sample from which the culture was isolated.

## TABLE I

Cultural Characteristics of *Saccharothrix espanaensis* LL-C19004

| Medium | Amount of Growth | Description of Colonial Growth | Soluble Pigments |
|---|---|---|---|
| Asparagine Dextrose Agar | Good | Flat waxy growth, 73. pale orange yellow to 75. deep yellowish brown | Brownish |
| Benedict's Agar | Moderate | Flat waxy growth, 70. light orange yellow to 71. moderate orange yellow | None |
| Bennett's Agar | Good to moderate | Flat waxy growth, 76. light yellowish brown to 77. moderate yellowish brown | None |
| Calcium Malate | Moderate | Flat waxy growth, 67. brilliant orange yellow; sparse white aerial mycelia | None |

TABLE I (continued)

| Medium | Amount of Growth | Description of Colonial Growth | Soluble Pigments |
|---|---|---|---|
| Gauze No. 1 Agar | Poor | Flat colorless colonies with a trace of white aerial mycelia | None |
| Glycerol Asparagine Agar | Moderate | Flat waxy growth, 73. pale orange yellow to 76. light yellowish brown; sparse white aerial mycelia | Yellowish |
| Glycerol Casein Agar | Moderate | Flat waxy growth, 90. grayish yellow; a trace of white aerial mycelia | None |
| Hickey-Tresner Agar | Moderate | Flat waxy growth, 79. light grayish yellow brown | None |
| Inorganic Salts-Starch Agar | Moderate | Flat waxy growth, 90. grayish yellow to 74. strong yellowish brown; trace white aerial mycelia | Yellow |

TABLE I (continued)

| Medium | Amount of Growth | Description of Colonial Growth | Soluble Pigments |
|---|---|---|---|
| NZ-amine Starch Glucose Agar | Good | Plicate ridged growth, 77. moderate yellowish brown to 75. deep yellowish brown | Brown |
| Oatmeal Agar | Moderate | Flat waxy growth, 79. light grayish yellow brown; white aerial mycelia | Brownish |
| Tomato Paste-Oatmeal Agar | Good | Plicate waxy growth, 86. light yellow to 77. moderate yellowish brown; no aerial mycelia | Brown |
| Yeast Extract-Malt Extract Agar | Good | Plicate waxy growth, 90. grayish yellow to 78. dark yellowish brown | Reddish brown |

TABLE II

Micromorphology of Saccharothrix espanaesis LL-C19004

| Medium | Aerial Mycelium and/or Sporiferous Structures | Spore Shape | Spore Size | Spore Surface |
|---|---|---|---|---|
| Tap water-crude agar | Rudimentary aerial mycelia, fragmenting into ovoid "spores"; vegetative mycelia also appear to fragment | ovoid | 0.6-0.8 micron x 1.0-1.2 micron | smooth |

TABLE III

Physiological Reactions of *Saccharothrix espanaensis* LL-C19004

| Medium | Incubation (Days) | Amount of Growth | Physiological Reaction |
|---|---|---|---|
| Peptone-Iron Agar | 7<br>14 | Good<br>Good | Slight browning<br>Slight browning |
| Tyrosine Agar (ISP-7) | 7<br>14 | Good<br>Good | Slight browning<br>Yellow pigment |
| Litmus Milk | 14<br>28 | Good<br>Good | Strong peptonization<br>Strong peptonization |
| Nutrient Gelatin | 14<br>28 | Good<br>Good | No proteolysis<br>No proteolysis |
| Nitrate Broth | 14<br>28 | Good<br>Good | No reduction<br>Weak reduction |
| Adenine Agar | 14<br>28 | Good<br>Good | Hydrolysis<br>Strong hydrolysis |
| Hypoxanthine Agar | 14<br>28 | Good<br>Good | Hydrolysis<br>Strong hydrolysis |
| Tyrosine Agar | 14<br>28 | Good<br>Good | No hydrolysis<br>No hydrolysis |
| Xanthine Agar | 14<br>28 | Good<br>Good | No hydrolysis<br>No hydrolysis |
| Urea Broth | 28 | Good | No decomposition |

TABLE III (continued)

| Medium | Incubation (Days) | Amount of Growth | Physiological Reaction |
|---|---|---|---|
| Esculin Broth | 14 | Good | Strong hydrolysis |
| NZ-Amine with Soluble Starch and Glucose Agar (ATCC Med. No. 172) | 5 | Poor or no growth at about 4°C and about 45°C; moderate growth at about 32°C and about 37°C; good growth at 28°C. | |

TABLE IV

Carbon Source Utilization of *Saccharothrix espanaensis* LL-C19004 on ISP-9 Carbohydrate Utilization Medium (Incubation: 28 days, about 28°C)

| Carbon Source | Utilization |
|---|---|
| Adonitol | 0 |
| l-Arabinose | 0 |
| Dulcitol | 0 |
| Fructose | 3 |
| d-Galactose | 2 |
| d-Glucose | 3 |
| Glycerol | 2 |
| i-Inositol | 0 |
| Lactose | 0 |
| Maltose | 2 |
| d-Mannitol | 0 |
| d-Mannose | 3 |
| d-Melezitose | 0 |
| d-Melibiose | 0 |
| d-Raffinose | 0 |
| l-Rhamnose | 0 |
| Salicin | 1 |
| Sorbitol | 0 |
| Sucrose | 3 |
| d-Trehalose | 3 |
| d-Xylose | 0 |
| Negative control | 0 |

3 = Good utilization
2 = Fair utilization
1 = Poor utilization
0 = No utilization

TABLE V

Acid Production from Various Carbohydrates by Saccharothrix espanaensis LL-C19004 on Gordon's Basal Inorganic Nitrogen Medium

Incubation: 28 days, about 28°C

| Carbon Source | Acid Production | |
|---|---|---|
| | 7 Days | 28 Days |
| Adonitol | - | - |
| l-Arabinose | - | - |
| Fructose | +/- | + |
| d-Galactose | - | + |
| d-Glucose | + | + |
| Glycerol | + | + |
| i-Inositol | - | - |
| Lactose | - | - |
| Maltose | + | + |
| d-Mannitol | - | - |
| d-Mannose | +/- | + |
| d-Melezitose | - | - |
| d-Melibiose | - | - |
| d-Raffinose | - | - |
| l-Rhamnose | - | - |
| Salicin | - | - |
| Sorbitol | - | - |
| Sucrose | +/- | + |
| d-Trehalose | +/- | + |
| d-Xylose | - | + |
| Negative control | - | - |

\+ = good production
\- = no production
+/- = some production

TABLE VI

Utilization of Organic Acids by *Saccharothrix espanaensis* LL-C19004 on Gordon's Modification of Koser's Basal Agar (Koser's Citrate Agar)

Incubation: 28 days, about 28°C

| Carbon Source | Utilization |
|---|---|
| Benzoate | - |
| Citrate | - |
| Lactate | + |
| Malate | + |
| Mucic Acid | - |
| Oxalate | - |

\+ = yes

\- = no

It is to be understood that for the production of this new antibacterial agent the present invention is not limited to this particular organism or to organisms fully answering the above growth and microscopic characteristics which are given for illustrative purposes only. In fact, it is desired and intended to include the use of mutants produced from this organism by various means such as exposure to X-radiation, ultraviolet radiation, N'-methyl-N'-nitro-N-nitrosoguanidine, actinophages and the like.

The *in vitro* antibacterial effects of antibiotic LL-C19004 were determined by standard agar dilution methods against clinical isolates obtained from medical centers representing various geographical areas in the United States. The inoculum of each culture was approximately 1 to 5 x $10^4$ colony forming units applied with the Steers multiple inocula replicator to plates containing the antibiotic in Mueller-Hinton agar. The agar was supplemented with about 57 sheep blood where required for the growth of the organism. Wilkins-Chalgren agar was used for testing the anaerobic bacteria. The results are presented in Table VII.

TABLE VII

*In vitro* Antibacterial Spectrum of Antibiotic LL-C19004

| Organisms (No. of Strains Tested) | | Minimal Inhibitory Concentration mcg/ml Range | Concentration mcg/ml Required to Inhibit 90% of Strains Tested |
|---|---|---|---|
| **Gram Positive** | | | |
| *Staphylococcus aureus* | (61) | 0.12-2 | 0.5 |
| *Streptococcus* spp. β-*hemolytic* | (11) | 1 -2 | 2 |
| *Streptococcus pneumoniae* | (11) | 0.5 -2 | 2 |
| *Streptococcus*(*enterococcus*) | (30) | 0.25-8 | 4 |
| **Gram Negative** | | | |
| *Neisseria gonorrhoeae* | (11) | 0.25-1 | 1 |
| *Haemophilus influenzae* | (11) | 1 -4 | 4 |
| *Escherichia coli* | (15) | 16 -32 | 32 |
| *Klebsiella* spp. | (15) | 16 -32 | 32 |
| *Enterobacter* spp. | (15) | 4 -64 | 64 |
| *Serratia* spp. | (15) | 32 ->128 | 32 |
| *Proteus* (indole+) spp. | (17) | 16 ->128 | >128 |
| *Acinetobacter* spp. | (15) | 4 -16 | 16 |
| *Pseudomonas aeruginosa* | (15) | 32 -128 | 128 |
| **Anaerobes** | | | |
| *Bacteroides fragilis* | (15) | >128 | >128 |

The *in vivo* activity of antibiotic LL-C19004 was assessed in female mice, strain CD-1, weighing 20± 2g each, infected intraperitoneally with sufficient bacterial cells suspended in broth or about 5% mucin to kill approximately 95 to 100 of untreated mice within about 48 hours. Antibiotic LL-C19004 was administered in single subcutaneous doses about 1/2 hour after infection. Seven day survival ratios from 3 to 4 separate tests were pooled for the determination of the median effective dose ($ED_{50}$) by probit analysis. The results are summarized in Table VIII.

TABLE VIII

In Vivo Activity of Antibiotic LL-C19004

| Infection | Median Effective Dose mg/kg (95% Confidence Limits) |
|---|---|
| Staphylococcus aureus Smith | 0.06 (0.04-0.07) |
| Staphylococcus aureus Rose | 2.6 (1.9 -3.5) |
| Streptococcus pyogenes C203 | 0.90 (0.70-1.1) |
| Streptococcus pneumoniae SV1 | 0.85 (0.70-1.1) |
| Escherichia coli No. 311 | >8 |
| Klebsiella pneumoniae AD | >8 |

General Fermentation Conditions

Cultivation of Saccharothrix espanaensis LL-C19004 may be carried out in a wide variety of liquid culture media. Media which are useful for the production of antibiotic LL-C19004 include an assimilable source of carbon, such as starch, sugar, molasses, glycerol, etc.; an assimilable source of nitrogen such as protein, protein hydrolysate, polypeptides, amino acids, cornsteep liquor, etc.; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the medium. Aeration in tanks and bottles is supplied by forcing sterile air through or onto the surface of the fermenting medium. Agitation is provided by a mechanical impeller. An antifoam agent may be added as needed. The growth of the organism is usually conducted at about 24-37°C, prefarably at about 28°C.

General Procedure for Isolation of Antibiotic LL-C19004

Antibiotic LL-C19004 is recovered from the fermentation filtrate by adsorption on a column of macroreticular resin such as Amberlite® IRC($NH_4^+$) and elution from the column with dilute alkali such as 2N ammonium hydroxide. The active fractions are further purified on a column of CM Sephadex® ($NH_4^+$) by elution with a water-1.5M ammonium hydroxide gradient.

Production of the antibiotic complex during the fermentation processing and purification steps can be followed by testing samples against an organism known to be sensitive to antibiotic LL-C19004. A turbidimetric bioassay procedure using Staphylococcus aureus ATCC 6538P grown in antibiotic medium No. 3 (Difco) and the Elanco Autoturb instrument is useful for this purpose.

Example 1

Inoculum Preparation

A typical medium used to grow the primary inoculum was prepared according to the following formula:

| | | |
|---|---|---|
| Dextrose.................. | about | 1.0% |
| Dextrin................... | about | 2.0% |
| Yeast extract............. | about | 0.5% |
| NZ Amine A................ | about | 0.5% |
| Calcium carbonate......... | about | 0.1% |
| Water..........qs......... | 100% | |

This medium was sterilized. An approximately 100 ml portion of this medium, in a 500ml flask, was inoculated with mycelial scrapings from an agar slant of the culture Saccharothrix espanaensis LL-C19004. The medium was then placed on a rotary shaker and agitated vigorously for approximately 72 hours at about 28°c, providing primary inoculum.

An approximate 100 ml portion of this primary inoculum was then used to inoculate about one liter of medium of the same formulation which was then incubated at about 28°Cwith aeration for approximately 48 hours, providing secondary inoculum.

Approximately one liter portion of this secondary inoculum was then used to inoculate about 26 liters of medium of the same formulation in a tank, which was incubated at about 28°C with agitation by an impeller driven at about 500 rpm, a sterile air flow of about 30 liters per minute and the addition of about 75 ml of a defoaming agent for about 48 hours, providing tertiary inoculum.

Example 2

Fermentation

A fermentation medium was prepared according to the following formulation:

| | | |
|---|---|---|
| Dextrin................... | about | 3.0 % |
| Molasses.................. | about | 2.0 % |
| Soya peptone.............. | about | 0.75% |
| Yeast extract............. | about | 0.25% |
| Water.......qs........... | 100 % | |

An approximate 245 liter portion of this medium in a tank was adjusted to pH 8.5-8.6, sterilized and then inoculated with about 30 liters of tertiary inoculum prepared as described in Example 1. Aeration was supplied at the rate of about 150 liters of sterile air per minute and agitation was supplied by an impeller driven at about 175 rpm. The temperature was maintained at about 28°C, silicone antifoam agent was added as required, and the fermentation was monitored for production of antibiotic LL-C19004 by antibacterial activity, TLC and HPLC analyses. The fermentation was terminated after about 80 hours, at which time the mash was harvested.

Example 3

Isolation of Antibiotic LL-C19004

The whole mash from three fermentations, conducted as described in Example 2, were pooled providing about 820 liters of mash. An approximate 41 kg portion of diatomaceous earth was added and this mixture was filtered, and the cake was washed with about 123 liters of water. The filtrate and wash were combined providing about 900 liters of solution at about pH 7.6 This solution was then passed, at a flow rate of 575-625 ml/minute, through a cloumn containing a 14 liter bed volume of Amberlite® IRC 50($NH_4^+$) ion exchange resin. The column was next washed with about 40 liters of water at a flow rate of 515-625 ml/minute and was then eluted with about 60 liters of 2N ammonium hydroxide, at a flow rate of 575-625 ml/minute, collecting fractions of about 6 liters each. Fraction number two was concentrated to about 2 liters

and then freeze-dried, providing about 114 g of solid.

An about 5.0 g portion of the above solid was mixed with about 25 ml of water, adjusted to about pH 7.0 with hydrochloric acid and placed on a 3 x 45 cm column of CM Sephadex® ($NH_4^+$ ). Pump pressure was applied to force the solution down the column. The column was eluted first with about 250 ml of water and then with a linear gradient of water-1.5M ammonium hydroxide for about 2 hours as about 5 ml fractions were collected. Pooling of fractions was based on an autoturb assay with Staphylococcus aureus 209P, ATCC No. 6538P. Fractions 108-119 were combined and freeze-dried, giving about 1.25 g of substantially pure antibiotic LL-C19004 as a white powder.

## Claims

**1.** Antibiotic LL-C19004, which:

a) has an approximate elemental analysis: C, 46.88; H, 6.93; N, 4.08; S, 1.79;
b) has ultraviolet absorption spectra as shown in FIG. 1;
c) has an infrared absorption spectrum as shown in FIG. 2;
d) has a proton magnetic resonance spectrum as shown in FIG. 3; and
e) has an optical rotation $[ \alpha ]_D^{25}$ = -85°±2°(c 0.5%, water), obtainable from Saccharothrix espanaensis (NRRL 15764).

**2.** A method for preparing a composition for treating bacterial infections in warm-blooded animals said composition comprising an antibacterially effective amount of LL-C19004 as defined in Claim 1.

**3.** A process for producing antibiotic LL-C19004, as defined in Claim 1, which comprises aerobically fermenting the microorganism Saccharothrix espanaensis (NRRL 15764) or mutants thereof in a liquid medium containing assimilable sources of carbon, nitrogen and inorganic salts, until substantial antibiotic activity is imparted to said medium and then recovering the antibiotic therefrom.

**4.** The process as recited in Claim 3 which comprises aerobically fermenting a liquid medium containing assimilable sources of carbon, nitrogen and inorganic salts, which medium has been inoculated with a viable culture or the microorganism Saccharothrix espanaensis (NRRL 15764) or mutants thereof, maintaining said fermentation culture at a temperature of about 24-32°C for a period of approximately 72-150 hours, harvesting the mash and extracting the antibiotic.

**5.** A biologically pure culture of the microorganism Saccharothrix espanaensis (NRRL 15764).

**6.** A biologically pure culture of the microorganism Saccharothrix espanaensis according to Claim 5, wherein said microorganism has spontaneously mutated, such that the microorganism is genetically altered but still retains the ability to synthesize antibiotic LL-C19004 as defined in Claim 1.

**7.** A biologically pure culture of the microorganism Saccharothrix espanaensis according to Claim 5, wherein said microorganism has been subjected to mutagenic means such that the microorganism is genetically altered but still retains the ability to synthesize antibiotic LL-C19004 as defined in Claim 1.

## Revendications

**1.** Antibiotique LL-C19004 qui:

a) a une analyse élémentaire approximative suivante : C, 46,88 ; H, 6,93 ; N, 4,08 ; S, 1,79 ;
b) a un spectre d'absorption d'ultraviolet montré à la figure 1 ;
c) a un spectre d'absorption d'infrarouge montré à la figure 2 ;
d) a un spectre de résonance magnétique du proton montré à la figure 3 ; et
e) a une rotation optique $[\alpha]_D^{25}$ = -85°±2°(c 0,5 %, dans l'eau), qui peut être obtenu à partir du Saccharothrix espanaensis (NRRL 15764).

**2.** Une méthode de préparation d'une composition pour traiter les infections bactériennes chez les animaux à sang chaud, ladite composition comprenant une quantité efficace de LL-C19004 tel que défini dans la revendication 1.

**3.** Un procédé de production de l'antibiotique LL-C19004, tel que défini dans la revendication 1, qui

comprend la fermentation aérobique du micro-organisme Saccharothrix espanaensis (NRRL 15764) ou des mutants de ce dernier dans un milieu liquide contenant des sources assimilables de carbone, d'azote et de sels inorganiques, jusqu'à ce qu'une activité antibiotique substantielle soit impartie audit milieu et ensutie la récupération de l'antibiotique à partir de ce milieu.

**4.** Le procédé selon la revendication 3, qui comprend la fermentation aérobique d'un milieu liquide contenant des sources assimilables de carbone, d'azote et de sels inorganiques, ledit milieu a été inoculé par une culture viable du micro-organisme Saccharothrix espanaensis (NRRL 15764) ou des mutants de ce dernier, le maintien de ladite culture en fermentation à une température d'environ 24-32°C pendant une période d'environ 72-150 h, la récolte du moût et l'extraction de l'antibiotique.

**5.** Une culture biologiquement pure du micro-organisme Saccharothrix espanaensis (NRRL 15764).

**6.** Une culture biologiquement pure du micro-organisme Saccharothrix espanaensis selon la revendication 5, selon laquelle ledit micro-organisme a été muté spontanément, de sorte que le micro-organisme est altéré génétiquement mais retient encore son aptitude à synthétiser l'antibiotique LL-C19004 tel que défini dans la revendication 1.

**7.** Une culture biologiquement pure du micro-organisme Saccharothrix espanaensis selon la revendication 5, selon laquelle ledit micro-organisme a été soumis à un moyen mutagène de sorte que le micro-organisme est altéré génétiquement mais retient encore son aptitude à synthétiser l'antibiotique LL-C19004 tel que défini dans la revendication 1.

**Patentansprüche**

**1.** Antibiotikum LL-C19004, welches:
(a) eine ungefähre Elementaranalyse: C 46,88; H 6,93; N 4,08; S 1,79 aufweist;
(b) Ultraviolett-Absorptionsspektren aufweist, wie in Figur 1 gezeigt;
(c) ein Infrarot-Absorptionsspektrum aufweist, wie in Figur 2 gezeigt;
(d) ein protonenkernmagnetisches Resonanzspektrum aufweist, wie in Figur 3 gezeigt; und
(e) eine optische Drehung $[\alpha]_D^{25} = -85^\circ \pm 2^\circ$ (c 0,5%, Wasser) hat, erhältlich von Saccharothrix espanaensis (NRRL 15764).

**2.** Verfahren zu Herstellung eines Mittels zur Behandlung von bakteriellen Infektionen bei warmblütigen Tieren, wobei das Mittel eine antibakteriell wirksame Menge des in Anspruch 1 definierte LL-C19004 umfasst.

**3.** Verfahren zur Herstellung von Antibiotikum LL-C19004 wie in Anspruch 1 definiert, umfassend die aerobische Fermentierung des Mikroorganismus Saccharothrix espanaensis (NRRL 15764) oder Mutanten desselben in einem flüssigen Medium, enthaltend assimilierbare Quellen von Kohlenstoff, Stickstoff und anorganischen Salzen, bis das Medium eine wesentliche antibiotische Aktivität aufweist und nachfolgend die Isolierung des Antibiotikum daraus.

**4.** Verfahren gemäß Anspruch 3, umfassend die aerobische Fermentierung eines flüssigen Mediums, enthaltend assimilierbare Quellen von Kohlenstoff, Stickstoff und anorganischen Salzen, wobei das Medium mit einer lebensfähigen Kultur des Mikroorganismus Saccharothrix espanaensis (NRRL 15764) oder Mutanten desselben inokkuliert wurde, das Halten der Fermentationskultur bei einer Temperatur von etwa 24 bis 32°C während eines Zeitraumes von etwa 72 bis 150 Stunden, das Ernten der Maische und das Extrahieren des Antibiotikum.

**5.** Biologisch reine Kultur des Mikroorganismus Saccharothrix espanaensis (NRRL 15764).

**6.** Biologisch reine Kultur des Mikroorganismus Saccharothrix espanaensis gemäß Anspruch 5, wobei der Mikroorganismus spontan mutiert ist, und zwar derart, daß der Mikroorganismus genetisch verändert ist, jedoch die Fähigkeit behalten hat, das Antibiotikum LL-C19004, wie es in Anspruch 1 definiert ist, zu synthetisieren.

**7.** Biologisch reine Kultur des Mikroorganismus Saccharothrix espanaensis gemäß Anspruch 5, wobei der

Mikroorganismus mutagenen Mitteln ausgesetzt wurde, derart, daß der Mikroorganismus genetisch verändert ist, jedoch die Fähigkeit behalten hat, das Antibiotikum LL-C19004, wie in Anspruch 1 definiert, zu synthetisieren.

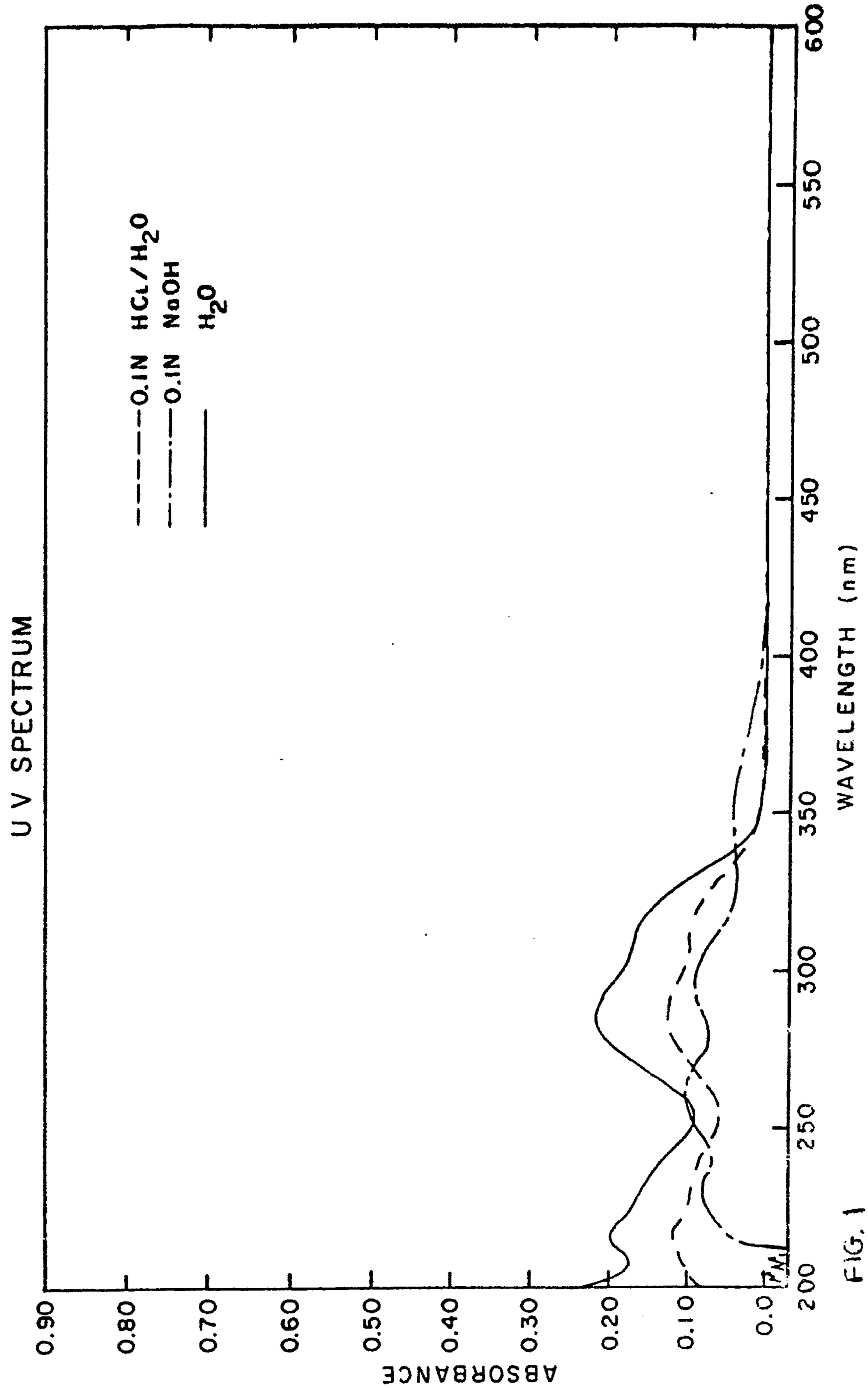
U V SPECTRUM
ABSORBANCE
0.90
0.80
0.70
0.60
0.50
0.40
0.30
0.20
0.10
0.0
WAVELENGTH (nm)
200
250
300
350
400
450
500
550
600
0.1N HCl/H2O
0.1N NaOH
H2O

FIG. 1

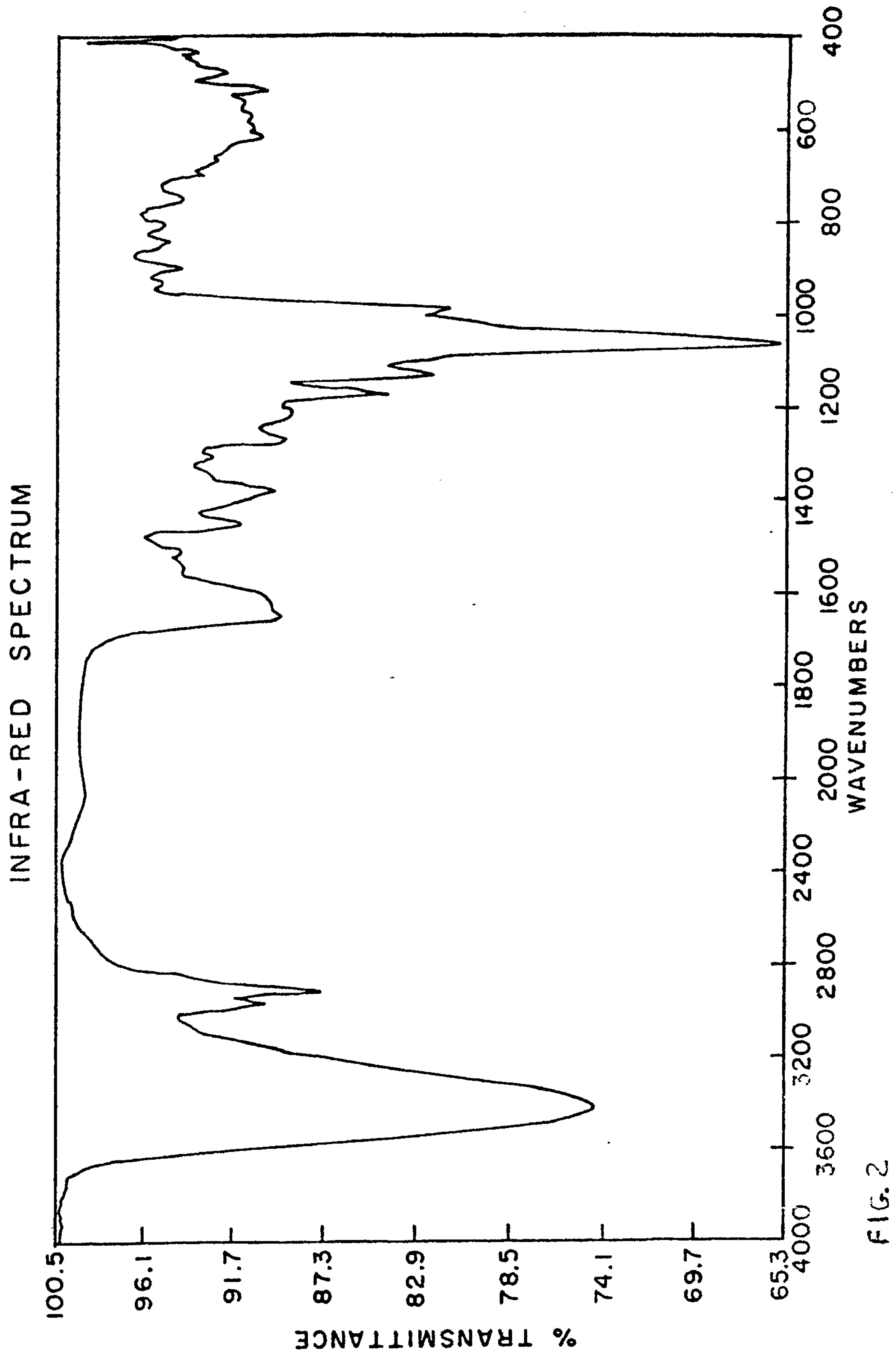
INFRA-RED SPECTRUM
% TRANSMITTANCE
100.5
96.1
91.7
87.3
82.9
78.5
74.1
69.7
65.3
WAVENUMBERS
4000
3600
3200
2800
2400
2000
1800
1600
1400
1200
1000
800
600
400

FIG. 2

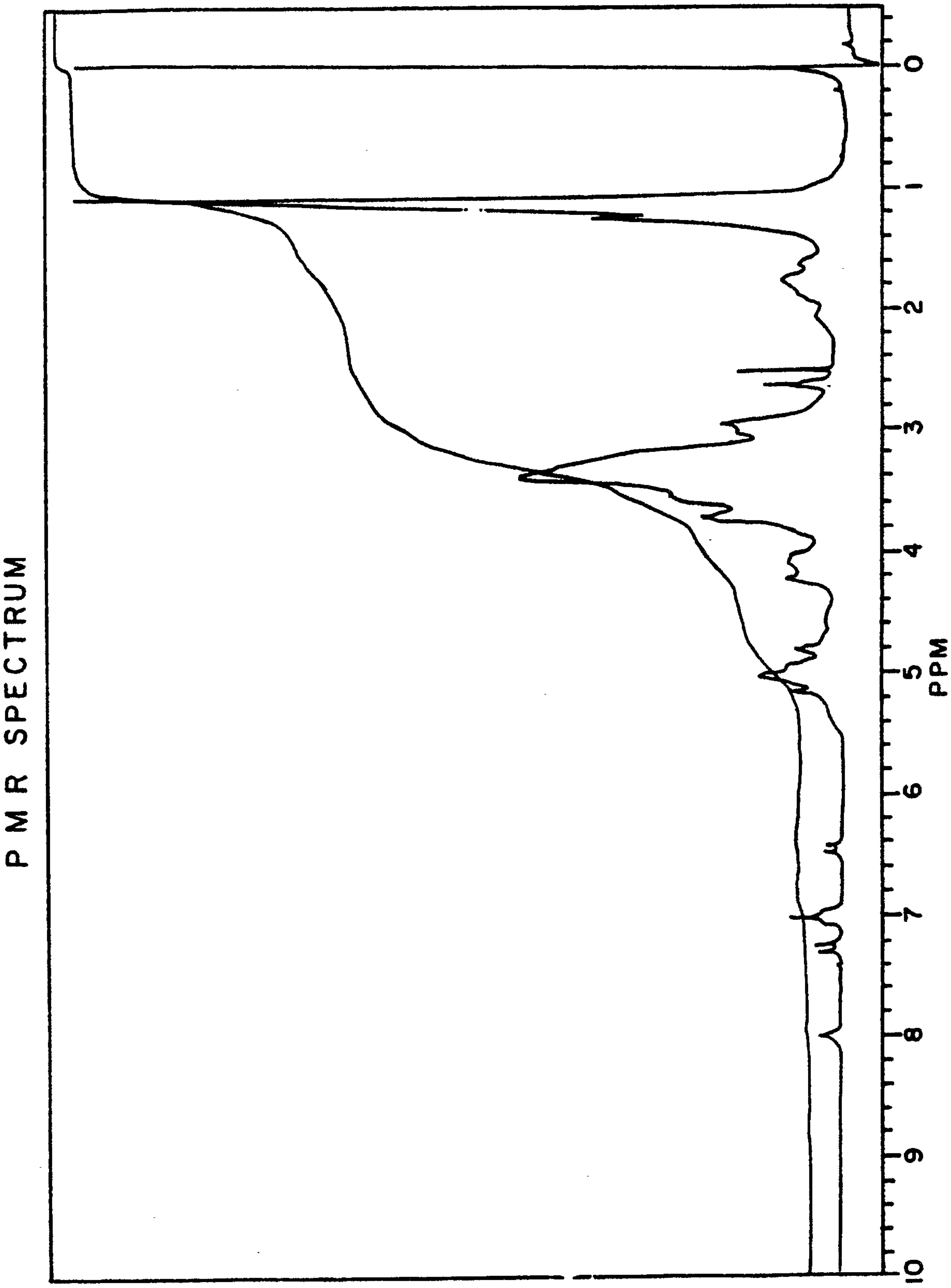


FIG. 3